# EUROPEAN PATENT SPECIFICATION

(11) **EP 3 471 610 B1**
(45) Date of publication and mention of the grant of the patent: **01.05.2024**
(21) Application number: 17729900.5
(22) Date of filing: 16.06.2017
(51) Int. Cl.: A61B 5/11

(54) **CARDIOVASCULAR AND CARDIORESPIRATORY FITNESS DETERMINATION**
QUANTITATIVE SEISMOKARDIOGRAFIE
SÉISMOCARDIOGRAPHIE QUANTITATIVE

(30) Priority: 16.06.2016 EP 16174743
(43) Date of publication of application: 24.04.2019
(73) Proprietor: Aalborg Universitet, 9220 Aalborg Øst (DK)
(72) Inventor: SCHMIDT, Samuel Emil, 9210 Aalborg SØ (DK); SØRENSEN, Kasper, 9000 Aalborg (DK); SØGAARD, Peter, 2300 København S (DK); STRUIJK, Johannes Jan, 9575 Terndrup (DK)
(74) Representative: Brann AB
(86) International application number: PCT/EP2017/064835
(87) International publication number: WO 2017/216375

(56) References cited:
- US-A1- 2006 276 849
- US-A1- 2011 263 994
- US-A1- 2013 231 576
- US-A1- 2015 038 856
- DI RIENZO M ET AL: "Wearable seismocardiography: Towards a beat-by-beat assessment of cardiac mechanics in ambulant subjects", AUTONOMIC NEUROSCIENCE: BASIC AND CLINICAL, ELSEVIER, AMSTERDAM, NL, vol. 178, no. 1, 9 May 2013 (2013-05-09), pages 50-59, XP028734850, ISSN: 1566-0702, DOI: 10.1016/J.AUTNEU.2013.04.005
- PANDIA KEYA ET AL: "A frequency domain analysis of respiratory variations in the seismocardiogram signal", ENGINEERING IN MEDICINE AND BIOLOGY SOCIETY (EMBC), 2013 34TH ANNUAL INTERNATIONAL CONFERENCE OF THE IEEE, IEEE, 3 July 2013 (2013-07-03), pages 6881-6884, XP032489567, ISSN: 1557-170X, DOI: 10.1109/EMBC.2013.6611139 [retrieved on 2013-09-25]

## Description

### Technical field of the Invention

The present invention generally relates to techniques for monitoring purposes relating to cardiovascular or cardiorespiratory fitness, and in particular to techniques for assisting in determining maximal oxygen consumption or uptake.

### Background of the Invention

Cardiovascular fitness and cardiorespiratory fitness refers to the ability of the circulatory and respiratory systems to supply oxygen to muscles. The latter term is generally used for the ability to supply oxygen to skeletal muscles during sustained physical activity, which may therefore be regarded as a subset of cardiovascular fitness.

These types of fitness are affected by physiological parameters, including heart rate, stroke volume, cardiac output, and maximal oxygen consumption. Regular exercise makes these systems more efficient by enlarging the heart muscle, enabling more blood to be pumped with each stroke, and increasing the number of small arteries in trained skeletal muscles, which supply more blood to working muscles.

There is both a clinical demand and a consumer demand for a low-cost and portable technology that can give an indication of cardiovascular and cardiorespiratory fitness.

Seismocardiography (SCG) is the analysis of sub-audible low-frequency vibrations at the chest wall caused by the beating heart. More generally, SCG typically relates to non-invasive measurement of accelerations in the chest wall produced by myocardial movement. Heart sounds are audible components of the chest wall vibrations that typically are above 40-60 Hz, while SCG vibrations typically are below 5 Hz.

SCG is typically measured using an accelerometer. However, when an accelerometer is used, both low frequency SCG components and audible components are simultaneously sampled. The SCG components and the audible components reveal different cardiovascular functions, thus enabling different approaches to diagnosing a cardiovascular function. For example, SCG is typically suitable for estimation of time intervals between features in the cardiac cycle, while heart sounds are appropriate for detection of murmurs caused by flow disturbances.

When using an accelerometer, the heart sounds or audio components in the accelerometer signal are dominated by the high intensity of the low-frequency vibrations, or SCG waves. If the accelerometer signal is high pass filtered, for example with a lower cutoff of 50 Hz, the heart sounds are revealed. In the heart sound, the most dominating sounds are the first heart sound (S1) and second heart sounds (S2), which are related to the mitral valve closure (MC) and the aortic valve closure (AC), respectively.

US2013231576 discloses a somatic data-measuring apparatus that can easily and accurately measure the optimal exercise intensity for the subject being measured, and a somatic data measurement method. The somatic data-measuring apparatus is provided with a heart sound-acquiring means that detects the subject's heart sounds and outputs same as heart sound data, a first heart sound-extracting means that detects the first heart sound on the basis of the heart sound data, a first heart sound amplitude-measuring means that measures the amplitude from the detected first heart sound and outputs same as first heart sound amplitude data, a heart rate-counting means that measures the subject's heart rate and outputs same as heart rate data, and an exercise intensity-computing means that computes the double product of the heart rate data and the first heart sound amplitude data as double product data and detects, as the optimal exercise intensity, the exercise intensity at which the approximation line, which approximates said double product data distribution, bends. Since the double product, which represents myocardial oxygen consumption, is effective as an index that accurately reflects the state of cardiac workload, it is possible to measure accurately the degree of workload on the heart.

US2006276849 discloses an implantable device and method for monitoring S1 heart sounds with a remotely located accelerometer. The device includes a transducer that converts heart sounds into an electrical signal. A control circuit is coupled to the transducer. The control circuit is configured to receive the electrical signal, identify an S1 heart sound, and to convert the S1 heart sound into electrical information. The control circuit also generates morphological data from the electrical information. The morphological data relates to a hemodynamic metric, such as left ventricular contractility. A housing may enclose the control circuit. The housing defines a volume coextensive with an outer surface of the housing. The transducer is in or on the volume defined by the housing.

### Object of the Invention

An object of the present invention is to meet the abovementioned demands of a technology that can give an indication of cardiovascular and cardiorespiratory fitness, and in particular a technology that is inexpensive and portable.

### Summary of the Invention

The invention is set out in the claims.

### Summary of the Disclosure

According to a first aspect, the aforementioned objects are accomplished by a method for quantifying, or determining an indication of, cardiovascular fitness or cardiorespiratory fitness. The method comprises: obtaining a plurality of segments of a signal recorded with an accelerometer placed on the chest of a person for measuring accelerations and vibrations of the chest wall of the person caused by myocardial movement, wherein each segment covers, or corresponds to, a cardiac cycle. The method further comprises: aligning the plurality of segments, determining a mean segment based on the plurality of segments, and filtering the plurality of segments prior to determining the mean segment, or filtering the mean segment, with a band-pass filter having a lower cutoff frequency below 1 Hz, and an upper cut-off frequency in the range 60-500 Hz. The method further comprises: determining a first temporal feature in the filtered mean segment, determining a measure based on at least one of the signal strength, or amplitude, of the first temporal feature and the location in time of the first temporal feature, and providing output information based on the determined measure.

Here, and throughout these specifications, quantifying, or determining an indication of cardiovascular fitness and cardiorespiratory fitness are understood to be limited to a normal function of the cardiovascular or cardiorespiratory system, and to be disassociated with, an abnormal cardiovascular or cardiorespiratory function, condition or structure, or a cardiovascular or cardiorespiratory disorder or disease. Thus, quantifying, or determining an indication of cardiovascular or cardiorespiratory fitness is understood to include quantifying, or determining an indication of, aerobic fitness, such as maximal oxygen consumption or uptake (VO₂ Max) .

Quantifying, or determining an indication of, cardiovascular fitness or cardiorespiratory fitness is understood to not include quantifying, or determining an indication, of function, such as cardiovascular function. Thus, quantifying, or determining an indication of, cardiovascular fitness or cardiorespiratory fitness is understood to not encompass quantifying, or determining an indication of, a heart disease relating to myocardial performance, such as heart failure. Here, function, or cardiovascular function, are understood to be limited to an abnormal cardiovascular or cardiorespiratory function, condition or structure, or a cardiovascular or cardiorespiratory disorder or disease, and to be disassociated with normal function of the cardiovascular or cardiorespiratory system.

Throughout these specifications, a temporal feature may correspond to a feature or stage in a cardiac cycle. A temporal feature may correspond to a peak, valley, local extremum, local minima, local maxima, maximal change, maximal increase, or maximal decrease of the filtered mean segment. A measure may, throughout these specifications, correspond to or be based on, a signal strength or an amplitude, or a difference in time. The signal strength or amplitude of a temporal feature may correspond to an acceleration affecting the accelerometer. Signal strength of a temporal feature is here, and throughout these specifications, understood to encompass a signal sample or a signal value of the temporal feature. The amplitude may be determined relative to the mean signal in the mean segment. An amplitude is understood to encompass a peak value, or the extreme value of a temporal feature, such as a local maxima or minima.

The accelerometer may comprise a piezoelectric element. The signal may represent a voltage generated by the piezoelectric element. Thus, the signal strength or amplitude of a temporal feature may represent a voltage value for the temporal feature.

According to a second aspect, the objects are achieved by system for quantifying, or determining an indication of, cardiovascular fitness or cardiorespiratory fitness. The system comprises:(A) an accelerometer configured to be placed on the chest of a person for measuring accelerations and vibrations of the chest wall of the person caused by myocardial movement, and (B) a processor operatively connected to the accelerometer. The processor is configured to: obtain a plurality of segments of a signal recorded with the accelerometer, wherein each segment covers, or corresponds to, a cardiac cycle. The processor is also configured to: align the plurality of segments, determine a mean segment based on the plurality of segments, and filter the plurality of segments prior to determining the mean segment, or filter the mean segment, with a band-pass filter having a lower cutoff frequency below 1 Hz and an upper cut-off frequency in the range 60-500 Hz. The processor is further configured to: determine a first temporal feature in the mean segment, determine a measure based on at least one of the signal strength, or amplitude, of the first temporal feature and the location in time of the first temporal feature, and provide output information based on the determined measure.

In the above aspects, to obtain a plurality of segments of a signal may comprise: obtaining the signal and forming the plurality of segments from the signal.

According to a third aspect, the objects are achieved by a system for quantifying, or determining an indication of, cardiovascular fitness or cardiorespiratory fitness. The system comprises: an accelerometer configured to be placed on the chest of a person for obtaining a signal representing accelerations and vibrations of the chest wall of the person caused by myocardial movement, and a segmentation module for forming a plurality of segments from the signal, wherein each segment covers, or corresponds to, a cardiac cycle. It further comprises: an align module for aligning the plurality of segments, a first calculation module for determining a mean segment based on the plurality of segments, and a filter module for filtering the plurality of segments prior to determining the mean segment, or for filtering the mean segment, with a band-pass filter having a lower cutoff frequency below 1 Hz and an upper cut-off frequency in the range 60-500 Hz. The system also comprises: a second calculation module for determining a first temporal feature in the mean segment and, a third calculation unit for determining a measure based on at least one of the signal strength, or amplitude, of the first temporal feature and the location in time of the first temporal feature, and an output module for providing output information based on the determined measure.

According to a fourth aspect, the objects are achieved by a computer program product for being used in a system for quantifying, or determining an indication of, cardiovascular fitness or cardiorespiratory fitness, wherein the system comprises: (A) an accelerometer for being placed on, or configured to be placed on, the chest of a person for measuring accelerations and vibrations of the chest wall of the person caused by myocardial movement, and (B) a processor operatively connected with the accelerometer. The computer program product comprising program code instructions configured to, when executed by the processor of the system, cause the processor to: obtain a signal with the accelerometer, and forming a plurality of segments from the signal, wherein each segment covers, or corresponds to, a cardiac cycle. The program code instructions further causes the processor to: align the plurality of segments, determine a mean segment based on the plurality of segments, and filter the plurality of segments prior to determining the mean segment, or filter the mean segment, with a band-pass filter having a lower cutoff frequency below 1 Hz and an upper cut-off frequency in the range 60-500 Hz. The program code instructions are further configured to cause the processor to: determine a first temporal feature in the mean segment, determine a measure based on at least one of the signal strength, or amplitude, of the first temporal feature and the location in time of the first temporal feature, and provide output information based on the determined measure.

According to a fifth aspect, the objects are achieved by a non-transient memory on which a computer program product according to the fourth aspect is stored.

According to a sixth aspect, the objects are achieved by a method for quantifying, or determining an indication of, cardiorespiratory fitness. The method comprises: obtaining a signal portion of a signal recorded with an accelerometer placed on the chest of a person for measuring accelerations and vibrations of the chest wall of the person caused by myocardial movement, determining a maximum value in the signal portion, and providing output information indicating cardiorespiratory fitness based on the maximum value.

Alternatively to determining a maximum value and providing output information on the maximum value, the method may comprise: determining the standard deviation or variance of the signal portion, and providing output information indicating cardiorespiratory fitness based on the standard deviation or variance.

The signal portion may be, or correspond to, a segment of the plurality of segments described in relation to the above aspects.

According to a seventh aspect, the objects are achieved by a system for quantifying, or determining an indication of, cardiorespiratory fitness. The system comprises:(A) an accelerometer configured to be placed on the chest of a person for measuring accelerations and vibrations of the chest wall of the person caused by myocardial movement, and (B) a processor operatively connected to the accelerometer. The processor is configured to perform any of the steps described in relation to the sixth aspect.

According to an eighth aspect, the objects are achieved by a system for quantifying, or determining an indication of, cardiorespiratory fitness. The system comprises: an accelerometer configured to be placed on the chest of a person for measuring accelerations and vibrations of the chest wall of the person caused by myocardial movement, a calculation module for obtaining a signal portion of a signal recorded with the accelerometer placed on the chest of a person, a determining module for determining a maximum value in the signal portion, and an output module for providing output information indicating cardiorespiratory fitness based on the maximum value.

According to a ninth aspect, the objects are achieved by a computer program product for being used in a system for quantifying, or determining an indication of, cardiorespiratory fitness. The system comprises:(A) an accelerometer configured to be placed on the chest of a person for measuring accelerations and vibrations of the chest wall of the person caused by myocardial movement, and (B) a processor operatively connected to the accelerometer. The computer program product comprising program code instructions configured to, when executed by the processor of the system, cause the processor to: perform any of the steps described in relation to the sixth aspect.

According to a tenth aspect, the objects are achieved by a non-transient memory on which a computer program product according to the ninth aspect is stored.

In the different aspects above, the output information may represent the actual determined measure. Alternatively, the output information may represent a score based on the determined measure. The output information may indicate, or be an indication of, cardiovascular fitness or cardiorespiratory fitness, or more precisely an indication of VO₂ Max, for example as one or more numerical values. Additionally or alternatively, the aligning may be performed prior to the filtering, and the filtering may be performed prior to determining the mean segment.

In the method of the first aspect, the accelerometer may be placed on the chest of a person and attached to the skin of the person by an adhesive for measuring the accelerations and vibrations. The systems of the second, third and fourth aspects may further comprise an adhesive patch configured for supporting the accelerometer and for being attached to the skin of the person. By attaching the accelerometer to the skin, the quality of the recorded signals is improved.

### Detailed description

The different aspects described above may be modified as described below.

The step of obtaining a segment of a signal recorded with an accelerometer may comprise: recording a signal with an accelerometer placed on the chest of a person for measuring accelerations and vibrations of the chest wall of the person caused by myocardial movement, wherein the signal is recorded over a period of time covering a plurality of cardiac cycles of the person. The step further may comprise: dividing the recorded signal into the plurality of segments, wherein each segment covers a single cardiac cycle. The accelerometer may be placed on the front of the chest of the person. The accelerometer being placed on the chest of a person means that it is placed on the outside and not on the inside of the body. This has the advantage of a simple application that does not require any chirurgical skills and that it can be performed in non-sterile environments.

Obtaining a plurality of segments of a signal recorded with an accelerometer may comprise: recording the signal with the accelerometer placed on the chest of a person for measuring accelerations and vibrations of the chest wall of the person caused by myocardial movement, and filtering the signal to obtain an audio signal. Obtaining a plurality of segments may further comprise: identifying a plurality of heart sounds in the audio signal, wherein each heart sound relates to a single cardiac cycle, and dividing the recorded signal into the plurality of segments based on the identified plurality of heart sounds. The filtering may comprise a high-pass filtering having lower cut-off frequency in the range 40-80 Hz, or approximately equal to 50 Hz or 65 Hz.

Here, the plurality of heart sounds may be the first heart sound (S1). Alternatively, the plurality of heart sounds may be the second heart sound (S2). Throughout these specifications, a microphone is understood as a transducer that converts sound into an electrical signal.

The aligning the plurality of segments may comprise: determining a heart sound in each of the plurality of segments, and aligning the plurality of segments by the determined heart sound of each segment. The heart sound may be the first heart sound (S1) or the second heart sound (S2). The first heart sound (S1) may correspond to the closing of the atrioventricular valves. The second heart sound (S2) may correspond to the closing of the semilunar valves.

The measure may correspond to, or be based on, the signal strength, or amplitude, of or at the first temporal feature. For example, the measure may correspond to the amplitude of the first heart sound (S1). This has been found to be an advantageous measure to study when examining cardiovascular fitness.

Further, determining a measure may comprise: determining the signal strength, or amplitude, of the first temporal feature. The first temporal feature may correspond to: the aortic valve opening (AO) of a heart cycle or the or the aortic valve closing (AC).

The method according to the first aspect may further comprise: determining a second temporal feature in the filtered mean segment, and wherein determining a measure is further based the signal strength, or amplitude, of the second temporal feature and on the location in time of the second temporal feature. By having two or more temporal features, additional measures can be used, thus contributing to an improved technology for determining cardiorespiratory fitness.

Alternatively or additionally, the measure may be based on the signal strength, or amplitude, of the first temporal feature and on the signal strength, or amplitude, of the second temporal feature. The signal strength, or amplitude, of the first temporal feature may be normalized by the signal strength, or amplitude, of the second temporal feature. Determining a measure may comprise: determining the difference or ratio between the signal strength, or amplitude, of the first temporal feature and the signal strength, or amplitude, of the second temporal feature, wherein the measure is based on the determined difference or ratio.

The first temporal feature may correspond to the aortic valve opening (AO) and the second temporal feature may correspond to the isovolumic movement (IM).

The method, or determining the first temporal feature, may further comprise: determining a first point in time in the mean segment corresponding to the onset of a heart sound. Determining the first temporal feature may further comprise: determining the first temporal feature relative to the first point in time. Similarly, determining the second temporal feature may further comprise: determining the second temporal feature relative to the first point in time. The heart sound may be the first heart sound (S1) or the second heart sound (S2). As mentioned above, the first heart sound (S1) may correspond to the closing of the atrioventricular valves and the second heart sound (S2) may correspond to the closing of the semilunar valves.

If the heart sound is the first heart sound (S1), determining a first temporal feature may comprise: determining the first local minima (IM) subsequent to the first point in time, and assigning the first local minima to represent the isovolumic movement (IM). Alternatively or additionally, determining a first temporal feature may comprise: determining the global maxima subsequent to the first point in time, and assigning the global maxima to represent the aortic valve opening (AO).

The lower cutoff frequency of the band-pass filter may be below 0.5 Hz, 0.2 Hz, or approximately 0.1 Hz. The upper cutoff frequency may be the range of 100-500 Hz, 150-250 Hz, 175-225 Hz, or approximately 200 Hz, or in one of the ranges 60-100 Hz, 100-150 Hz, 150-200 Hz, 200-250 Hz, and 250-300 Hz. According to the invention, the upper cutoff frequency is in the range of 60-500 Hz, according to embodiments not covered by the claimed invention it may also be in the range 30-500 Hz. These frequencies for providing the SCG signal have been found to give reliable results.

The method may further comprise: determining a heart rate of the beating heart. Similarly, the processor may be configured to: determine a heart rate of the beating heart, and the computer program product may comprising program code instructions configured to, when executed by the processor of the system, cause the processor to: determine a heart rate of the beating heart. The heart rate may be determined based on the signal recorded with the accelerometer. The heart rate may indicate the number of contraction of the heart per minute or another suitable period of time.

Determining the measure may further be based on the heart rate. For example, determining the measure may comprise: determining the difference between the location in time of a first temporal feature and the location in time of a second temporal feature and dividing the difference with the heart rate. It is contemplated that by taking the heart rate into account, the measure can be determined more accurately for persons having a high heart rate at rest and for persons that are active or exercising when the signal is recorded with the accelerometer.

In the method of the sixth aspect, obtaining a signal portion may comprises: recording a signal with an accelerometer placed on the chest of a person, and forming the signal portion from the signal, wherein the signal portion covers one or more complete cardiac cycles of the person.

The method according to the sixth aspect may further comprise: determining a minimum value in the signal portion, and the output information indicating cardiorespiratory fitness may further be based on the difference between the maximum value and the minimum value. The maximum value may correspond to the peak of a first temporal feature in a cardiac cycle. Similarly, the minimum value may correspond to the peak of a second temporal feature in a cardiac cycle.

Here, the first temporal feature and the second temporal feature may belong to the same cardiac cycle. The first temporal feature may follow immediately after the second temporal feature, and/or the peak of the first temporal feature may be within 100 ms of the peak of the second temporal feature. Alternatively, the first temporal feature and the second temporal feature may belong to different cardiac cycles.

The method according to the sixth aspect may further comprise: determining the minimum value and the maximum value within a time interval having a length that is less than 100 ms. The maximum value, or the first temporal feature, may correspond to the signal strength, or amplitude, of the aortic valve opening (AO). The minimum value, or the second temporal feature, may correspond to the signal strength, or amplitude, of the isovolumic movement (IM). The maximum value and/or the minimum value may be a peak amplitude value. Effectively, this means that the maximum value corresponds to the peak value of the aortic valve opening (AO), and the minimum value corresponds to the peak value of the isovolumic movement (IM).

The method according to the sixth aspect may further comprise: obtaining information indicating the Body Mass Index (BMI) of the person, and the output information indicating cardiorespiratory fitness may further be based on the indication of BMI. It has been found that there is a strong correlation between BMI and the indication of VO₂ Max, and that the indication can be corrected for BMI. It is contemplated that this correlation is an effect of fat dampening vibrations of the of the chest wall caused by myocardial movement.

The method according to the invention further comprises : filtering the signal portion with a band-pass filter having a lower cutoff frequency below 1 Hz and an upper cut-off frequency in the range 60-500 Hz. The lower cutoff frequency of the band-pass filter may be below 0.5 Hz, 0.2 Hz, or approximately 0.1 Hz. The upper cutoff frequency may be the range of 100-400, 150-250 Hz, 175-225 Hz, or approximately 200 Hz, or in one of the ranges 60-100 Hz, 100-150 Hz, 150-200 Hz, 200-250 Hz, and 250-300 Hz.

The upper cutoff frequency is in the range of 60-500 Hz, according to embodiments not covered by the claimed invention it may also be in the range 30-500 Hz. These frequencies for providing the SCG signal have been found to give reliable results. The signal portion may cover one complete cardiac cycle, less than ten cardiac cycles, less than 30 cardiac cycles, or less than 60 cardiac cycles. The signal portion may have a length that is less than 10 seconds, 30 seconds, or 60 seconds.

The systems of the above aspects may comprise a non-transient memory storing program code instructions that, when executed by the processor, configures the processor to perform the described steps and/or have the described functions.

The systems may comprise a smart-phone. The processor and/or the non-transient memory may be integral parts of the smart-phone. Further, the accelerometer may be an integral part of the smart-phone. The system may also comprise a casing or holder for supporting the smart-phone, and the casing or holder may comprise an adhesive patch configured for attaching the casing or holder to the skin of the person. Alternatively to the accelerometer being an integral part of the smart-phone, the accelerometer may form part of an auxiliary unit configured to communicate with the smart-phone by wire or wirelessly, such as a fitness band that can be strapped around the chest of person.

Providing the output information may further comprise: storing the plurality of segments, the mean segment, the signal portion, and/or the measure in the non-transient memory or in an auxiliary non-transient memory. The auxiliary non-transient memory may form part of computer server system, which may be at a remote location.

Providing the output information may further comprise: providing a previously obtained measure and the output information may further be based on the previously obtained measure. The previously obtained measure may be stored in the non-transient memory or in the auxiliary non-transient memory. The output information may be based on the difference between the measure and the previously obtained measure. For example, the output information may be the difference in amplitude between an amplitude of the aortic valve opening (AO) and a previously obtained amplitude of the aortic valve opening (AO).

Additionally or alternatively, providing the output information may further comprise: providing a previously obtained mean segment, or signal portion, and the output information may further be based on the mean segment, or signal portion, and the previously obtained mean segment, or signal portion. The previously obtained mean segment, or signal portion, may be stored in the non-transient memory or in the auxiliary non-transient memory. The output information may comprise: a graph overlying the mean segment, or signal portion, with the previously obtained mean segment, or signal portion. More specifically, the output information may comprise: a graph overlying a portion of the mean segment and the corresponding portion of the previously obtained mean segment. For example, the portion may cover the first heart sound (S1). The graph may be displayed on the screen of abovementioned smart-phone.

The previously obtained measure or previously obtained mean segment, or signal portion, may have been determined in the same manner as the measure or the mean segment, or by the same steps as performed for determining the measure or the mean segment. The previously obtained measure or previously obtained mean segment, or signal portion, may have been determined at an earlier point in time, such as more than five days, ten days, or eight weeks prior to determining the measure, the mean segment, or the signal portion. This is particularly advantageous when studying how cardiovascular fitness changes during an extended period of training or exercising.

Further advantages with and features of the different aspects will be apparent from the following description of the drawing.

### Brief description of the drawings

A more complete understanding of the abovementioned and other features and advantages of the present invention will be apparent from the following detailed description of the drawings, wherein:
Fig.1 is a schematic illustration of an embodiment of a system 12 for quantifying cardiovascular or cardiorespiratory fitness,
Fig.2 is a flow chart illustrating the basic steps of a method employed in the system described in relation to Fig.1,
Fig.3 is a flow chart illustrating sub-steps and additional steps of a method based on the method described in relation to Fig.2,
Figs.4a and 4b shows a segment that has been filtered by a band-pass filter and to a high-pass filter, respectively,
Fig.5 is a schematic illustration of an embodiment of a system an alternative embodiment for quantifying the function of a beating heart, and
Fig.6 is a flow chart illustrating the steps of an alternative method employed in the system described in relation to Fig.1.

### Detailed description of drawings

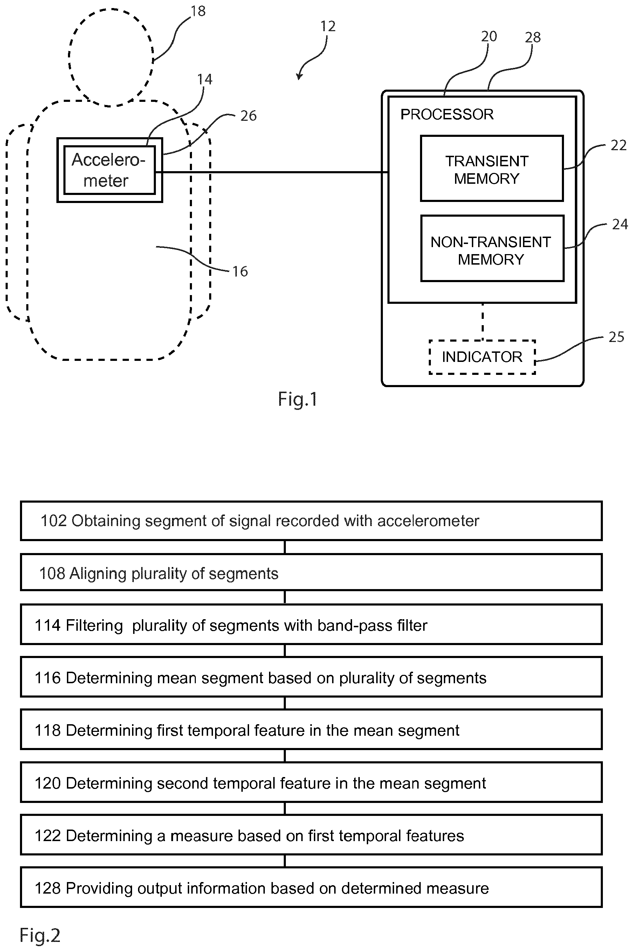

Fig.1 schematically illustrates an embodiment of a system 12 for quantifying cardiovascular or cardiorespiratory fitness. The system 12 has an accelerometer 14 in the form of a piezoelectric element that can be placed on the chest of a person 18 and for measuring vibrations of the chest wall caused by movements of the heart. A processor 20 is connected with the accelerometer 14. The processor 20 has a transient memory 22 which can store a signal received from the accelerometer 14, and by which it can execute program code instructions. The system 12 comprises a support 26 that supports the accelerometer 14 and a housing 28 that accommodates the processor 20. The system 12 also has a non-transient memory 24 storing program code instructions for the processor 20. For example, the system 12 as a whole can be an integral part of a smart-phone, or all parts except the accelerometer 20 and the support 26 can form part of a smart-phone. In one embodiment, the accelerometer is an integrated accelerometer of a smart-phone.

In one embodiment of the system 12, it additionally has an indicator 25 operatively connected with the processor 20. The indicator 25 can, for example, have an LCD display, or the like, that can display output information from the processor 20, such as a number.

The program code instructions in the non-transient memory cause the processor 20 to perform a method that is shown in Fig.2. The accelerometer is placed on the chest of a person and a signal is recorded. A plurality of segments is then obtained 102, where each segments corresponds to a heartbeat or a cardiac cycle. This is followed by an alignment 108 and a filtering 114. Here, a band-pass filter is employed having a lower cut-off frequency of approximately 0.1 Hz, and an upper cut-off frequency of approximately 200 Hz. Subsequently, a mean segment is determined 118 from the plurality of segments.

With the mean segment formed, a temporal feature is determined 120. The temporal feature in turn is used to determine 122 a measure. Examples of temporal features and measures are described below. Output information is then provided 128 based on the determined measure. In one embodiment, the output information is a number that is displayed on the abovementioned indicator 25.

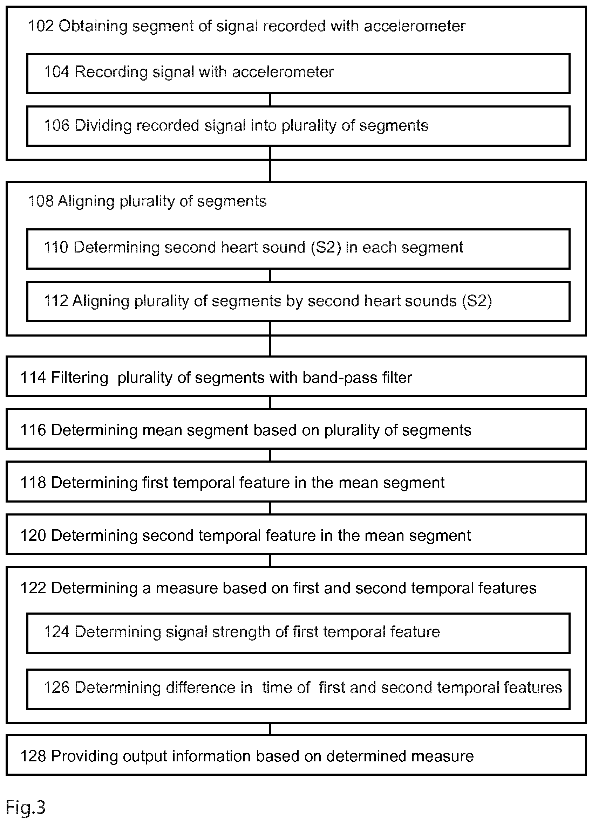

Further details of the method are shown in the flow chart of Fig.3. The step of obtaining 102 the plurality of segments includes the sub-steps of recording 104 the signal with the accelerometer, and dividing 106 the recorded signal into the plurality of segments, for example by a technique as described in US 8235912 B2 and US 8469896 B2 relying on audible components of the accelerometer signal.

In an alternative embodiment, an electrocardiography (ECG) signal is acquired simultaneously to the accelerometer signal, and the ECG signal is used for the segmentation of the latter. For example, a segmentation as described in Jensen et al. (Computing in Cardiology 2014; 41:29-32) can be used.

When obtaining 102 the plurality of segments, a method similar to the method described in Jensen et al. is employed to remove noisy segments. A high-pass filter with a lower cut-off of 65 Hz is applied to the segments and the onset of the first heart sound S1 is then determined by a known technique. Similarly, a high-pass filter with a lower cut-off of 50 Hz is applied to the segments and the onset of the second heart sound S2 is then determined by a known technique. The segments are then aligned according to the determined second heart sound S2. In alternative embodiment, the first heart sound is used instead.

The mean segment is determined 116 by summing the aligned segments to a single segment and dividing the resulting signal strength, or amplitude, by the number of segments in the sum.

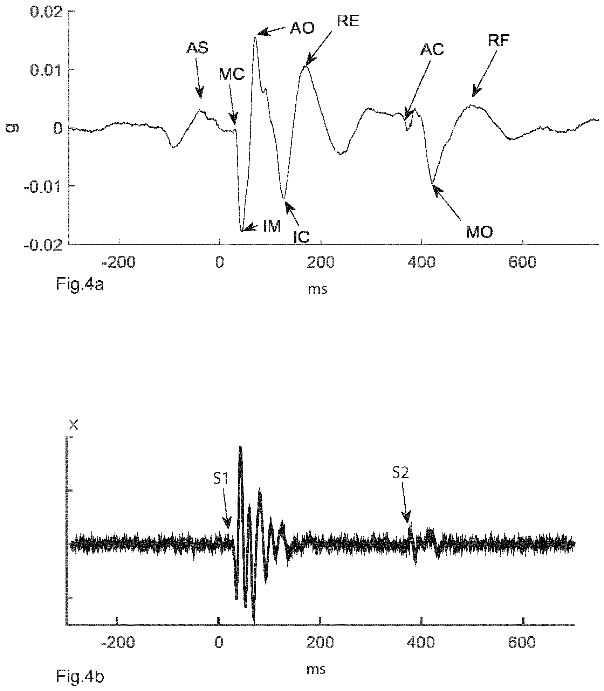

Fig.4a shows a segment that has been subjected to the above described band-pass filtering. The abscissa represents an acceleration in g (ms⁻²) and the ordinate the time in milliseconds (ms). Here, g is proportional to the voltage from the accelerometer 14. The zero point of the ordinate corresponds to the R peak in a simultaneously recorded segment of an ECG signal. A number of temporal features are indicated in Fig.4a, which are further described below.

Fig.4b shows a segment that has been subjected to a high-pass filter with a lower cut-off of 50 Hz, as described above. The abscissa represents the signal strength X (no unit) and the ordinate the time in milliseconds (ms). The latter has been aligned by the simultaneously recorded segment of an ECG signal in the same manner as described in relation to Fig.4a. The onset of the first heart sound (S1) and the second heart sound (S2) are indicated in Fig.4b.

In the steps of determining 118 the first temporal feature and determining 120 the second temporal feature 120 in the mean segment, the following temporal features are identified in the mean segment: the isovolumic movement (IM), the aortic valve opening (AO), and the aortic valve closure (AC) .

For example, with the first point in time determined to be the onset of the first heart sound (S1), see Fig.4b, the isovolumic movement (IM) is determined as the first local minima (IM) subsequent to the first point in time and the aortic valve opening (AO) is determined as the global maxima subsequent to the first point in time. For example, the first point in time can be determined by a technology similar to the technology described in US 8235912 B2 and US 8469896 B2. In an alternative embodiment, the isovolumic movement (IM) is determined as the global minima and the aortic valve opening (AO) is determined as the global maxima of the mean segment.

Measures or values are determined for the amplitudes or signal strengths of the aortic valve opening (AO) and aortic valve closure (AC) is determined, and the difference in amplitudes or signal strengths of the aortic valve opening (AO) and the isometric contraction (IM).

Output is then provided 128 in the form of values that are displayed on an LCD display of the indicator 206, where the values represents the determined signal strengths and differences in time in the examples above.

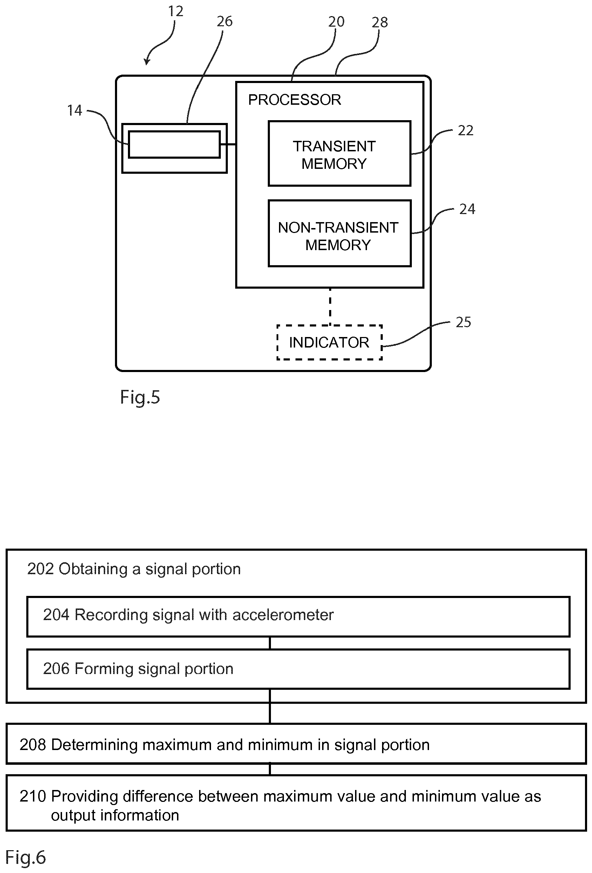

Fig.5 illustrates an alternative embodiment of the system 12 described in relation to Fig.1, with the only difference that the support 26 forms part of the housing 28 such that the housing 28 covers at least a portion of the accelerometer 14. In this embodiment, the housing 28 is placed on the chest of a person, which means that the accelerometer 14 is also placed on the chest of a person.

Fig.6 is a flow chart illustrating the steps of an alternative method employed in the systems described in relation to Figs.1 or 5. The program code instructions in the non-transient memory cause the processor 20 to perform a method that is shown in Fig.6. The accelerometer 14 is first placed on the chest of a person and a signal portion is first obtained 202 by recording 204 a signal with the accelerometer 14. The signal portion is then formed 206 from the signal covering a complete cardiac cycle. A band-pass filter is employed to the signal portion having a lower cut-off frequency of approximately 0.1 Hz, and an upper cut-off frequency of approximately 200 Hz.

The maximum value in the signal portion is then determined 206. Effectively, the maximum value corresponds to the amplitude of the aortic valve opening (AO), or a first temporal feature, which can be seen in Fig.4a. In one embodiment not illustrated in Fig.6, the maximum value is provided as output information indicating cardiorespiratory fitness.

The minimum value in the signal portion is also determined 208. Effectively, the minimum value corresponds to the amplitude of the isovolumic movement (IM), or a second temporal feature.

The maximum value corresponds to the peak of a first temporal feature in a cardiac cycle. Similarly, the minimum value corresponds to a peak of a second temporal feature in a cardiac cycle. The first temporal feature and the second temporal feature belong to the same cardiac cycle. The first temporal feature follows immediately after the second temporal feature, and the peak of the first temporal feature is within 100 ms of the peak of the second temporal feature, as can be seen in Fig.4a.

Output information indicating cardiorespiratory fitness is then provided 120 as the difference between the maximum value and the minimum value.

In an alternative embodiment, the system comprises an interface for inputting information to the processor. The method then also includes the step of obtaining information indicating the BMI of the person via the interface, and the output information indicating cardiorespiratory fitness is further be based on the indication of BMI, for example as part of a correction for BMI.

### Proof of concept

A custom lightweight 8 g piezoelectric accelerometer was developed for acquisition of the SCG signals. The low weight provides a better signal and the miniaturization allows for the accelerometer to be incorporated in another device. The accelerometer was used in a system as described above in relation to Figs.1-4. It should be noted that the system identifies AO, IM, and AC.

The system was used on a group of 17 untrained females undergoing a fitness program for an 8 week period. All subjects were tested before and after entering the program with a traditional VO₂ Max test (including ECG recordings) and a SCG recording at rest. The effect of the exercise program was significant by increasing the mean VO₂ Max from 28.7 ml/min/kg to 31.2 ml/min/kg (p=0.002). VO₂max was increased in 13 (76%) out the 17 subjects.

The study shows a strong correlation between VO₂ Max and SCG measures exemplified in the table below showing that the SCG is qualified for determining the cardiorespiratory fitness of a person.

| Amplitude | Correlation coefficient | P-value |
|---|---|---|
| peakAO - peakIM | 0.62 | 0.0001 |
| peakAC - bottomAC | 0.71 | 0.0000025 |
| AO | 0.58 | 0.00035 |
| BMI | -0.7 | 0.0000039 |

Table 1 shows the correlation of the selected features to VO₂ Max. Here, peakAO is the maximum value of the AO feature, peakIM is the minimum value of the IM feature, peakAC the maximum value of the AC feature, and bottomAC is the minimum value of the AC feature. It is contemplated that the difference peakAO - peakIM corresponds to the peak-to-peak amplitude of the AO and IM features, and that the difference peakAC - bottomAC corresponds the peak-to-peak amplitude AC feature. BMI is the Body Mass Index.

### Feasible modifications of the Invention

The invention is not limited only to the embodiments described above in relation to the drawings, which primarily have an illustrative and exemplifying purpose. This patent application is intended to cover all adjustments and variants of the preferred embodiments described herein, thus the present invention is defined by the wording of the appended claims . Thus, the equipment may be modified in all kinds of ways within the scope of the appended claims.

## Claims

1. A method for quantifying, or determining an indication of, cardiorespiratory fitness comprising:
- recording a signal with an accelerometer placed on the chest of a person, the accelerometer being configured for measuring accelerations and vibrations of the chest wall of the person caused by myocardial movement,
- forming a signal portion from the signal, wherein the signal portion covers one or more complete cardiac cycles of the person,
- filtering the signal portion with a band-pass filter having a lower cutoff frequency below 1 Hz and an upper cut-off frequency in the range 60-500 Hz,
- determining a maximum value in the signal portion, and
- providing output information indicating cardiorespiratory fitness based on the maximum value.

2. The method according to claim 1 wherein the band-pass filter has a lower cutoff frequency below 0.5 Hz, 0.2 Hz, or approximately 0.1 Hz.

3. The method according to any of claims 1-2 wherein the band-pass filter has an upper cut-off frequency in the range of 100-400 Hz, 150-250 Hz, or 175-225 Hz, or in one of the ranges 60-100 Hz, 100-150 Hz, 150-200 Hz, 200-250 Hz, and 250-300 Hz.

4. The method according to any of claims 1-3 further comprising:
- determining a minimum value in the signal portion, and wherein the output information indicating cardiorespiratory fitness is further based on the difference between the maximum value and the minimum value.

5. The method according to claim 4, wherein the maximum value corresponds to the peak of a first temporal feature in a cardiac cycle and the minimum value corresponds to the peak of a second temporal feature in a cardiac cycle.

6. The method according to claim 5, wherein the first temporal feature and the second temporal feature belong to the same cardiac cycle.

7. The method according to claim 6, wherein the first temporal feature follows immediately after the second temporal feature, and/or the peak of the first temporal feature is within 100 ms of the peak of the second temporal feature.

8. The method according to claim 5, wherein the first temporal feature and the second temporal feature belong to different cardiac cycles.

9. The method according to any of the claims 4-8 further comprising:
- determining the minimum value and the maximum value within a time interval having a length that is less than 100 ms.

10. The method according to any of the claims 1-9, wherein the maximum value, or the first temporal feature, corresponds to the signal strength, or amplitude, of the aortic valve opening (AO).

11. The method according to any of the claims 4-10, wherein the minimum value, or the second temporal feature, corresponds to the signal strength, or amplitude, of the isovolumic movement (IM).

12. The method according to any of the claims 4-9, wherein the maximum value, or the first temporal feature, corresponds to the maximum amplitude of the aortic valve closure (AC), and the minimum value, or the second temporal feature, corresponds to the minimum amplitude of the AC.

13. The method according to any of the claims 1-12 further comprising:
- obtaining information indicating the Body Mass Index, BMI, of the person, and the output information indicating cardiorespiratory fitness is further be based on the indication of BMI.

14. A system (12) for quantifying, or determining an indication of, cardiorespiratory fitness, comprising:
(A) an accelerometer (14) configured to be placed on the chest of a person for measuring accelerations and vibrations of the chest wall of the person caused by myocardial movement, and
(B) a processor (20) operatively connected to the accelerometer,
wherein the processor is configured to perform any of the steps of the method according to claims 1-13.

15. A computer program product for being used in a system for quantifying, or determining an indication of, cardiorespiratory fitness, wherein the system comprises:(A) an accelerometer configured to be placed on the chest of a person for measuring accelerations and vibrations of the chest wall of the person caused by myocardial movement, and (B) a processor operatively connected to the accelerometer, wherein the computer program product comprising program code instructions configured to, when executed by the processor of the system, cause the processor to: perform any of the steps of the method according to claims 1-13.

16. A non-transient memory on which a computer program product according to claim 15 is stored.

## Patentansprüche

1. Verfahren zum Quantifizieren oder Bestimmen eines Hinweises auf die kardiorespiratorische Fitness, umfassend:
- Aufzeichnen eines Signals mit einem Beschleunigungsmesser, der auf der Brust einer Person platziert ist, wobei der Beschleunigungsmesser zum Messen von Beschleunigungen und Vibrationen der Brustwand der Person konfiguriert ist, die durch Myokardbewegungen hervorgerufen werden,
- Bilden eines Signalabschnitts aus dem Signal, wobei der Signalabschnitt einen oder mehrere vollständige Herzzyklen der Person abdeckt,
- Filtern des Signalabschnitts mit einem Bandpassfilter mit einer unteren Grenzfrequenz unter 1 Hz und einer oberen Grenzfrequenz im Bereich 60-500 Hz,
- Bestimmen eines Maximalwerts in dem Signalabschnitt und
- Bereitstellen von Ausgabeinformationen, die die kardiorespiratorische Fitness basierend auf dem Maximalwert angeben.

2. Verfahren nach Anspruch 1, wobei der Bandpassfilter eine untere Grenzfrequenz unter 0,5 Hz, 0,2 Hz oder ungefähr 0,1 Hz aufweist.

3. Verfahren nach einem der Ansprüche 1-2, wobei der Bandpassfilter eine obere Grenzfrequenz im Bereich von 100-400 Hz, 150-250 Hz oder 175-225 Hz oder in einem der Bereiche 60-100 Hz, 100-150 Hz, 150-200 Hz, 200-250 Hz und 250-300 Hz aufweist.

4. Verfahren nach einem der Ansprüche 1-3, ferner umfassend:
- Bestimmen eines Minimalwerts im Signalabschnitt, und wobei die Ausgabeinformationen, die die kardiorespiratorische Fitness angeben, ferner auf der Differenz zwischen dem Maximalwert und dem Minimalwert basieren.

5. Verfahren nach Anspruch 4, wobei der Maximalwert dem Spitzenwert eines ersten zeitlichen Merkmals in einem Herzzyklus entspricht und der Minimalwert dem Spitzenwert eines zweiten zeitlichen Merkmals in einem Herzzyklus entspricht.

6. Verfahren nach Anspruch 5, wobei das erste zeitliche Merkmal und das zweite zeitliche Merkmal zum gleichen Herzzyklus gehören.

7. Verfahren nach Anspruch 6, wobei das erste zeitliche Merkmal unmittelbar nach dem zweiten zeitlichen Merkmal folgt und/oder der Spitzenwert des ersten zeitlichen Merkmals innerhalb von 100 ms vom Spitzenwert des zweiten zeitlichen Merkmals liegt.

8. Verfahren nach Anspruch 5, wobei das erste zeitliche Merkmal und das zweite zeitliche Merkmal zu verschiedenen Herzzyklen gehören.

9. Verfahren nach einem der Ansprüche 4-8, ferner umfassend:
- Ermitteln des Minimalwerts und des Maximalwerts innerhalb eines Zeitintervalls mit einer Länge, die kleiner als 100 ms ist.

10. Verfahren nach einem der Ansprüche 1-9, wobei der Maximalwert oder das erste zeitliche Merkmal der Signalstärke oder Amplitude der Aortenklappenöffnung (AO) entspricht.

11. Verfahren nach einem der Ansprüche 4-10, wobei der Minimalwert oder das zweite zeitliche Merkmal der Signalstärke oder Amplitude der isovolumischen Bewegung (IM) entspricht.

12. Verfahren nach einem der Ansprüche 4-9, wobei der Maximalwert oder das erste zeitliche Merkmal der maximalen Amplitude des Aortenklappenverschlusses (AC) entspricht und der Minimalwert oder das zweite zeitliche Merkmal der minimalen Amplitude des AC entspricht.

13. Verfahren nach einem der Ansprüche 1-12, ferner umfassend:
- Erhalten von Informationen, die den Body-Mass-Index (BMI) der Person angeben, und die Ausgabeinformationen, die die kardiorespiratorische Fitness angeben, ferner auf der Angabe des BMI basieren.

14. System (12) zum Quantifizieren oder Bestimmen eines Hinweises auf die kardiorespiratorische Fitness, umfassend:
(A) einen Beschleunigungsmesser (14), der dazu konfiguriert ist, auf der Brust einer Person platziert zu werden, um Beschleunigungen und Vibrationen der Brustwand der Person zu messen, die durch Myokardbewegungen hervorgerufen werden, und
(B) einen Prozessor (20), der betriebsfähig mit dem Beschleunigungsmesser verbunden ist, wobei der Prozessor dazu konfiguriert ist, einen der Schritte des Verfahrens nach den Ansprüchen 1-13 auszuführen.

15. Computerprogrammprodukt zur Verwendung in einem System zum Quantifizieren oder Bestimmen einer Angabe der kardiorespiratorischen Fitness, wobei das System Folgendes umfasst: (A) einen Beschleunigungsmesser, der dazu konfiguriert ist, auf der Brust einer Person platziert werden kann, um Beschleunigungen und Vibrationen der Brustwand der Person zu messen, die durch eine Myokardbewegung hervorgerufen werden, und (B) einen Prozessor, der betriebsfähig mit dem Beschleunigungsmesser verbunden ist, wobei das Computerprogrammprodukt Programmcodeanweisungen umfasst, die dazu konfiguriert sind, bei Ausführung durch den Prozessor des Systems den Prozessor zu Folgendem zu veranlassen: Ausführen eines beliebigen der Schritte des Verfahrens nach den Ansprüchen 1-13.

16. Nichtflüchtiger Speicher, auf dem ein Computerprogrammprodukt nach Anspruch 15 gespeichert ist.

## Revendications

1. Procédé pour quantifier, ou déterminer une indication de, la capacité cardiorespiratoire comprenant :
- l'enregistrement d'un signal avec un accéléromètre placé sur la poitrine d'une personne, l'accéléromètre étant configuré pour mesurer les accélérations et les vibrations de la paroi thoracique de la personne provoquées par le mouvement du myocarde,
- la formation d'une partie de signal à partir du signal, la partie de signal couvrant un ou plusieurs cycles cardiaques complets de la personne,
- le fait de filtrer la partie de signal avec un filtre passe-bande ayant une fréquence de coupure inférieure à 1 Hz et une fréquence de coupure supérieure dans la plage de 60 à 500 Hz,
- la détermination d'une valeur maximale dans la partie de signal, et
- la fourniture d'informations de sortie indiquant une capacité cardiorespiratoire sur la base de la valeur maximale.

2. Procédé selon la revendication 1, dans lequel le filtre passe-bande a une fréquence de coupure inférieure à 0,5 Hz, 0,2 Hz ou environ 0,1 Hz.

3. Procédé selon l'une quelconque des revendications 1 et 2, dans lequel le filtre passe-bande a une fréquence de coupure supérieure dans la plage de 100 à 400 Hz, 150 à 250 Hz ou 175 à 225 Hz, ou dans l'une des plages de 60 à 100 Hz, 100 à 150 Hz, 150 à 200 Hz, 200 à 250 Hz et 250 à 300 Hz.

4. Procédé selon l'une quelconque des revendications 1 à 3, comprenant en outre :
- la détermination d'une valeur minimale dans la partie de signal, et dans lequel les informations de sortie indiquant la capacité cardiorespiratoire sont en outre basées sur la différence entre la valeur maximale et la valeur minimale.

5. Procédé selon la revendication 4, dans lequel la valeur maximale correspond au pic d'une première caractéristique temporelle dans un cycle cardiaque et la valeur minimale correspond au pic d'une seconde caractéristique temporelle dans un cycle cardiaque.

6. Procédé selon la revendication 5, dans lequel la première caractéristique temporelle et la seconde caractéristique temporelle appartiennent au même cycle cardiaque.

7. Procédé selon la revendication 6, dans lequel la première caractéristique temporelle suit immédiatement la seconde caractéristique temporelle, et/ou le pic de la première caractéristique temporelle est à moins de 100 ms du pic de la seconde caractéristique temporelle.

8. Procédé selon la revendication 5, dans lequel la première caractéristique temporelle et la seconde caractéristique temporelle appartiennent au même cycle cardiaque.

9. Procédé selon l'une quelconque des revendications 4 à 8, comprenant également :
- la détermination de la valeur minimale et de la valeur maximale dans un intervalle de temps ayant une longueur inférieure à 100 ms.

10. Procédé selon l'une quelconque des revendications 1 à 9, dans lequel la valeur maximale, ou la première caractéristique temporelle, correspond à l'intensité de signal, ou à l'amplitude, de l'ouverture de valve aortique (AO).

11. Procédé selon l'une quelconque des revendications 4 à 10, dans lequel la valeur minimale, ou la seconde caractéristique temporelle, correspond à l'intensité de signal, ou à l'amplitude, du mouvement isovolumétrique (IM).

12. Procédé selon l'une quelconque des revendications 4 à 9, dans lequel la valeur maximale, ou la première caractéristique temporelle, correspond à l'amplitude maximale de la fermeture de valve aortique (AC), et la valeur minimale, ou la seconde caractéristique temporelle, correspond à l'amplitude minimale de l'AC.

13. Procédé selon l'une quelconque des revendications 1 à 12, comprenant en outre :
- l'obtention d'informations indiquant l'indice de masse corporelle, IMC, de la personne, et les informations de sortie indiquant la capacité cardiorespiratoire sont en outre basées sur l'indication de l'IMC.

14. Système (12) pour quantifier, ou déterminer une indication de, la capacité cardiorespiratoire comprenant :
(A) un accéléromètre (14) configuré pour être placé sur la poitrine d'une personne pour mesurer les accélérations et les vibrations de la paroi thoracique de la personne provoquées par le mouvement du myocarde, et
(B) un processeur (20) connecté de manière fonctionnelle à l'accéléromètre,
dans lequel le processeur est configuré pour exécuter l'une quelconque des étapes du procédé selon les revendications 1 à 13.

15. Produit programme informatique destiné à être utilisé dans un système pour quantifier, ou déterminer une indication de, la capacité cardiorespiratoire, où le système comprend : (A) un accéléromètre configuré pour être placé sur la poitrine d'une personne pour mesurer les accélérations et les vibrations de la paroi thoracique de la personne provoquées par le mouvement du myocarde, et (B) un processeur connecté de manière fonctionnelle à l'accéléromètre, le produit programme informatique comprenant des instructions de code de programme configurées pour, lorsqu'elles sont exécutées par le processeur du système, amener le processeur à : mettre en œuvre l'une quelconque des étapes du procédé selon les revendications 1 à 13.

16. Mémoire non transitoire sur laquelle un produit programme informatique selon la revendication 15 est stocké.
